# EUROPEAN PATENT SPECIFICATION

(11) **EP 1 971 275 B1**
(45) Date of publication and mention of the grant of the patent: **25.11.2009**
(21) Application number: 07716393.9
(22) Date of filing: 09.01.2007
(51) Int. Cl.: A61F 2/00

(54) **HERNIA PATCH FRAME**
BRUCHPFLASTERRAHMEN
CADRE DE PIÈCE DE RÉPARATION DES HERNIES

(30) Priority: 12.01.2006 US 758445 P
(43) Date of publication of application: 24.09.2008
(73) Proprietor: Minnesota Medical Development Inc., Plymouth, MN 55447 (US)
(72) Inventor: BROWN, Roderick, D., Glenwood, MN 56334 (US); AFREMOV, Michael, St. Louis Park, MN 55423 (US)
(74) Representative: Charig, Raymond Julian
(86) International application number: PCT/US2007/000329
(87) International publication number: WO 2007/087146

(56) References cited:
- EP-A- 0 544 485
- EP-A- 1 491 159
- WO-A-02/35990
- US-A- 5 824 082
- US-A- 5 824 082
- US-A1- 2004 087 980
- US-A1- 2005 228 486
- US-A1- 2005 228 486
- US-B1- 6 669 735

## Description

**Field of the Invention:** This invention relates generally to apparatus for use in hernia repair surgery, and more particularly to a prosthetic hernia repair patch that can be collapsed into a tube for laparoscopic delivery through a trocar and which deploys to a generally planar form when ejected from the trocar into the abdominal cavity.

**Discussion of the Prior Art:** Laparoscopic hernia repair procedures have been successfully used to treat inguinal, ventral, incisional and umbilical hernias for many years. Patients who utilized such laparoscopic procedures typically have recovered faster and undergone less pain than those who have undergone open surgery.

In the past, when carrying out early laparoscopic hernia procedures, initially a prosthetic patch was made at the time of surgery from a sterile, woven, polypropylene mesh material. This material was folded in half to create a double layer and then cut to size, typically a 6 cm by 9 cm rectangle. Sutures were used to join the four corners of the rectangle and two additional sutures were positioned approximately midway along the unfolded edge. A slit was then created between these two additional sutures which was designed to accommodate the inferior epigastric vessels. Following dissection of the hernia sac away from the ipsilateral testicle and cord structures, the creation of a peritoneal incision and the subsequent dissection of the peritoneal flap and hernia sac away from the hernia defect and surrounding fascia and cord structures, the patch was rolled into a tube and inserted into a trocar sleeve that was then introduced through a larger diameter trocar, and delivered into the peritoneal cavity. A laparoscopic forceps was then used to unfurl the patch and place it anterior to the hernia defect and around the inferior epigastric vessels with the mesh covering both the direct and indirect hernia spaces. The mesh patch would then be held in place by stapling or suturing it to underlying fascia. Subsequently, the peritoneum was closed over the patch so that the entire piece of mesh was covered thereby. While the above procedure proved quite successful in terms of outcomes, the need to fabricate the mesh patch at the time of surgery, the later difficulty in unrolling and positioning the mesh patch material relative to the direct and indirect hernia spaces, and the need to then staple or suture the mesh patch in place necessarily added significantly to the time and expense required for carrying out the procedure. Hernia patches were thereafter developed for use in laparoscopic surgery that were prefabricated to conform to anatomical structures, as disclosed in U.S. Pat. No. 5,824,082 to Brown. These patches generally made use of an outer wire frame of an alloy having shape memory properties, and a prosthetic fabric material that attached about its periphery to this frame. The device could be readily deployed when released from a tubular laparoscopic introducer and remained in place without a need for stapling or suturing to underlying facie. This patch design, however, is somewhat difficult to manufacture as the mesh material utilized in its design required extensive stitching around its periphery to hold the wire or cable frame in place against the mesh. Therefore, a need exists for a hernia patch for use in laparoscopic surgery, which uses a simplified design that overcomes past difficulties of manufacture. The present invention fulfills that need.
EP 0544485 describes a tissue repair device comprising a circular piece of barrier material kept spread, mainly in a plane shape by a thin Nitinol wire stiffener The stiffener is formed as an elongate flattened loop with a central fastening means for attachment to the banter material. US 2005/0228486 describes a medical implantable device with two zig-zag frame members with leaflets sutured thereto. US 1491159. US 6669735 and US 5824082 describe implantable prostheses and WO 02/35990 describes a supported lattice for cell cultivation.

### SUMMARY OF THE INVENTION

The hernia repair patch of the present invention comprises a wire frame of various designs including, but not limited to, a pair of overlapping "V" shaped wire segments with vertices disposed in opposite directions and further containing attached points at the free ends of the wire segments and a closed or slightly open circular loops near the vertices of both the "V" shaped wires. A synthetic prosthetic material, such as woven polypropylene or expanded PTFE (Gortex), is attached to the wire frame at only the few attached points although the material generally is supported by the entire wire frame. The wire frame supporting the mesh material may be formed of Nitinol or other suitable shape memory alloy. The frame is attached to the prosthetic material to apply a stretching force to the material. The device may have a generally rectangular shape of mesh with rounded corners when the alloy is in its austenite form and a compact shape of low profile when in a martensite form.

The shape memory property of Nitinol may be either stress-induced or temperature-induced. When temperature-induced, the atomic percent of nickel in the alloy is such that the alloy exhibits a transformation temperature at about 37°C (body temperature). Thus, when the patch is cooled, it can be readily formed into a compact configuration for placement in a delivery trocar. When ejected out of the trocar into the patient's abdominal cavity, the patch warms to the point where the alloy is in its austenite form so that it springs into its functional, predetermined configuration. The central portion of the patch accommodates the inferior epigastric vessels and cord structures while the opposed end lobes cover the direct and indirect hernia space. Because the frame is integral to the patch, it does not migrate and, accordingly, need not be sutured or stapled in place. It remains anchored following its being covered by the peritoneum in a sandwich or laminated fashion.

The foregoing features, objects and advantages of the invention will become apparent to those skilled in the art from the following detailed description of a preferred embodiment, especially when considered in conjunction with the accompanying drawings.

### DESCRIPTION OF THE DRAWINGS

Figure 1 is an enlarged plan view of the hernia repair patch of the present invention;
Figure 2 is an enlarged plan view of the hernia repair patch in a collapsed configuration; and
Figure 3 is an enlarged view of one of the wires used to hold the frame member in place before attachment to the mesh material.
Figure 4 is an enlarged view of an alternative embodiment of one of the wires used to hold the frame member in place before attachment to the mesh material.

### DESCRIPTION OF THE PREFERRED EMBODIMENT

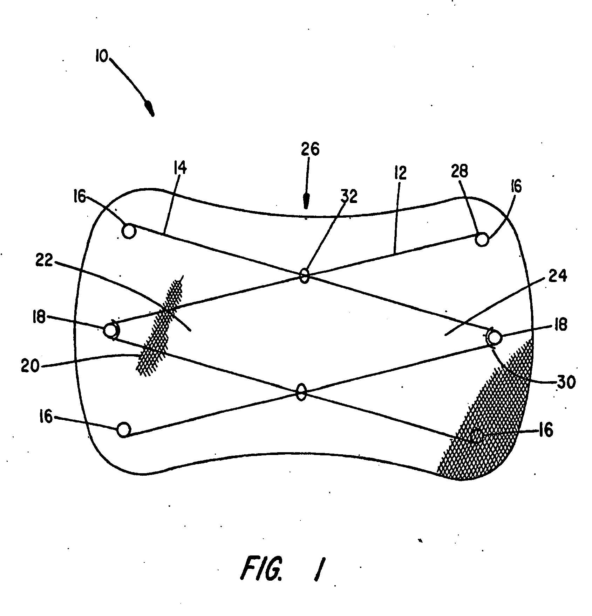

Referring first to Figure 1, there is shown an enlarged view of the hernia repair patch of the present invention. The patch device is generally indicated by numeral 10 and includes an outer frame of wire members 12 and 14. These wire members are preferably made up of a shape memory alloy of nickel and titanium commonly referred to as Nitinol. The shape memory properties of Nitinol can be especially useful in this device. For example, by proper adjustment of the relative concentration of nickel in the alloy, the wire frame can be made to exhibit a transition between austenitic form to martensitic form at about body temperature, thereby allowing for transformation to a convenient shape for either delivery or use.

Alternatively, by proper adjustment of the relative concentration of nickel in the alloy, the wire frame can be made to exhibit a transition between austenitic form and martensitic form based upon stress-induced shape memory properties. In this case, a small amount of stress is placed on the Nitinol wire for deformation during delivery of the device before the wire regains its shape upon deployment and removal of that stress, irrespective of temperature. Although Nitinol wire (2-2.5mm diameter) is preferred, wire members 12 and 14 may also be made of stainless steel, medical grade plastic, or other suitable material exhibiting a resilient property. The frame wires 12 and 14 are preferably radiopaque. While not utilizing the shape memory properties of Nitinol, these devices can be sufficiently collapsed and deployed if desired.

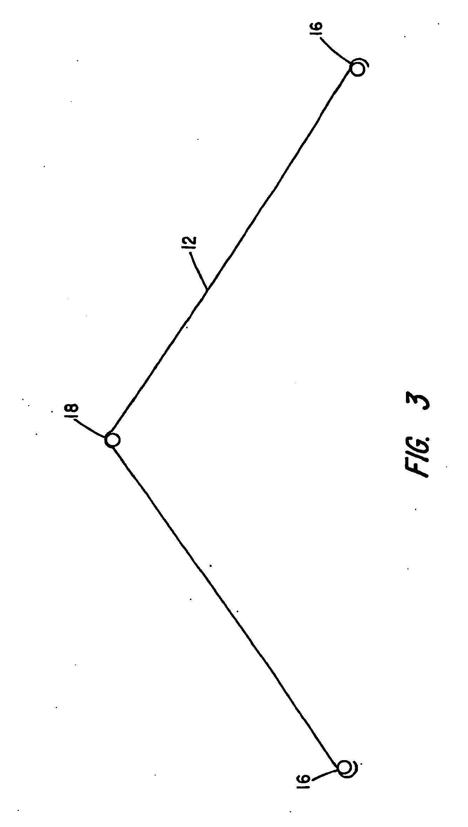

Wire members 12 and 14 are separate, overlapping "V" shaped wire segments disposed in inverse orientations. The two ends of each wire are attach points 16. At the attach points 16, the wire may be bent in a circular, rounded fashion such that closed loops of approximately 540 degrees are formed. Near the vertices 18 of the wire frame members, they are bent in a rounded fashion so as to form either a closed loop of about 540 degrees or a slightly open loop where the two sides of the wire converge. Both wire frame members 12 and 14 have sides of unequal length where one side is typically 5-10mm shorter than the other. This design allows for a slimmer profile when the device is collapsed for delivery, as will be discussed later. Generally, the manufactured shape of the wire is originally formed with a wide angle of 90-120 degrees between the two sides of the wire. (See Figure 3) This dimension is narrowed to about 30 degrees when the wire is being attached to the mesh member so as to provide more spring expansion with a slimmer wire.

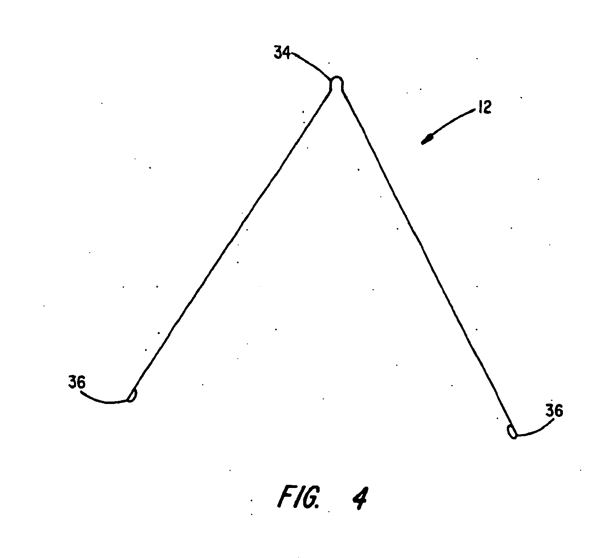

Alternatively, wire member 12 may be configured as in Fig. 4. Here, the wire member 12 includes a u-shaped apex 34. Whereas in the preferred embodiment shown in Figures 1-3 the vertices 18 of the wires are coiled loops, in this alternative embodiment the apex 34 is u-shaped and uses a suture loop to secure it to the mesh while allowing some slippage. Furthermore, the attachment points 36 are circular or slightly oval-shaped loops as opposed to the circular coils used in the preferred embodiment. The attachment points 36 use a suture loop to secure the mesh to the wire member. In the alternative embodiment, as in the preferred embodiment, the wire member has sides of unequal lengths.

Supported on the wire members 12 and 14 is a prosthetic fabric 20, preferably woven of polypropylene plastic of expanded PTFE (Gortex). The overall shape of the fabric is rectangular with rounded corners. There may also be slight indentations along the upper and lower borders of this mesh, although this is optional. Various sizes of mesh could be used with wire lengths adjusted accordingly. This material allows the device to be steam sterilized. When placed in a patient's body, the two sides of fabric mesh 20, referred to as lobes 22 and 24, are adapted to be positioned over the direct and the indirect hernia spaces, respectively. When this is done, the central portion at the intersection of the wire members 12 and 14, indicated generally by numeral 26, will allow placement without interference with the inferior epigastric vessels.

The manner in which the fabric is held to the wires differs significantly from prior art devices. First, at each of the four attach points 16 of the wires, elastic suture material 28 is used to hold the fabric mesh 20 to the wires 12 and 14. Using elastic sutures helps to accommodate elongation as the wire frame members are squeezed closed to pass through a cannula during insertion of the device into a body. The curvatures 18 at the vertices of the wires use a suture loop 30 to secure mesh while allowing slippage. Allowing this slippage and expansion of the vertices 18 of the wire member is necessary as these midpoints tend to expand outward when squeezed together. When the device is constructed in this way, there is a substantial savings of time and effort to manufacture the prosthetic as only six fastening points are required at the ends and midpoints, rather than the hundred or so used to secure mesh to its frame in some prior art devices. The simple design and easy to manufacture construction make for an extremely useful device.

Additionally, or alternatively, slipping the loops at the ends and midpoints of the members 12 and 14 over one or two strands of mesh can facilitate attachment to the mesh. This operates in a similar fashion to slipping a key in to a key ring. Attaching mesh this way eliminates the need for using any type of fastening sutures and reduces manufacturing and assembly costs. Also, loops 32 of polypropylene or similar thread-like material are preferably placed around wires 12 and 14 at their two cross over points and through the mesh for wire stabilization. These would be loose enough to allow the wire to slide through it when compressed for delivery.

For additional comfort and protection of a patient, the mesh material 20 is folded over the somewhat pointed ends of the frame members and secured to make the ends of the wire 16 less traumatic.

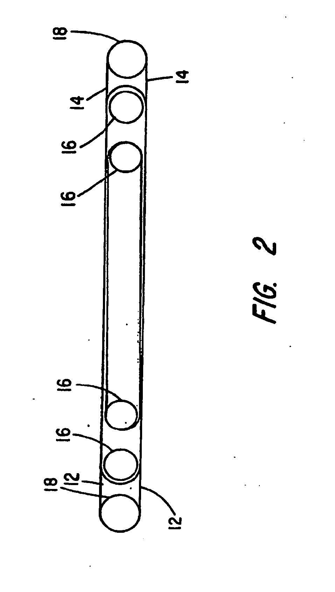

Assuming members 12 and 14 are Nitinol and that they are cooled below the transformation state so that they are in their martensitic form, the prosthesis 10 can be collapsed in an accordion-type manner to form a generally cylindrical structure as illustrated in Figure 2. Note the way that a slim profile results, facilitating delivery due to the rounded ends of the frame wires 12 and 14 being located at various locations as best seen in Figure 2.

Typically, the outside diameter of the attach points and vertices 16 and 18 would be around 3mm to facilitate fitting though a 5mm cannula when the mesh is attached. This collapsed shape allows the prosthesis to be introduced into the abdominal cavity through a tubular trocar. As the shape memory alloy members 12 and 14 warm up to body temperature, they transform to their austenitic form as shown in Figure 1. Using a laparoscopic forceps, the prosthesis 10 of Figure 1 can be grasped and repositioned by the surgeon until the lobes 22 and 24 and the narrowed center section 26 are appropriately located for covering the hernia defect. Observing this placement of the device is possible as the device is visible when viewed on an x-ray machine.

Those skilled in the art will appreciate that the prosthesis may be manufactured in a variety of shapes and sizes to accommodate children, adults, males and females and especially the type of hernia encountered. It can be contained in a sterile pack until ready for use. While Nitinol is the preferred shape memory alloy, other alloys, such as gold-cadnuum, nickel-aluminum and manganese-copper would also be suitable. Moreover, the prosthetic fabric material 20 need not be polypropylene mesh, but can also comprise other suitable materials, such as body compatible biaxially oriented polymeric films.

Without limitation, the major axis of the prosthesis 10 is typically in a range of about 8-16 cms long and the minor axis typically is about 5-12 cms wide. Such device can be tightly fitted into a cylinder, as shown in Figure 2, so as to fit within the internal lumen of a trocar or introducer sheath.

The invention has been described herein in considerable detail in order to comply with the patent statutes and to provide those skilled in the art with the information needed to apply the.novel principles and to construct and use such specialized components as are required. However, it is to be understood that the invention can be carried out by specifically different equipment and devices, and that various modifications, both as to the equipment and operating procedures, can be accomplished without departing from the scope of the claims.

## Claims

1. A hernia repair patch (10) comprising:
(a) a wire frame (12, 14) formed from a shape memory alloy wherein the wire frame includes two overlapping v-shaped wire segments (12, 14) each having sides unequal in length and with vertices (18) thereof disposed in opposite directions, wherein the wire segments have attach points (16) disposed on free ends of each of the wire segments and at their vertices, the attach points disposed on the free ends comprising closed loops;
(b) a prosthetic fabric material (20) attached to and supported by said frame wherein suture stitches (28, 30) hold the fabric to the wire frame at said attach points, wherein ` the wire frame applies a stretching force to the fabric material.

2. The hernia repair patch of claim 1 where the attach points at the vertices (18) are formed by bending the wire segments (12, 14) to form said loops.

3. The hernia repair patch of claim 2 where each loop is about 540 degrees.

4. The hernia repair patch of claim 1 wherein the angle between the two sides of the v-shaped wire segments (12, 14) is approximately between 90 and 120 degrees when in a relaxed state and compressed to about 30 degrees when the wire is attached to the fabric material.

5. The hernia repair patch of claim 1 wherein loops (32) of thread are placed around the wire segments (12, 14) where the wire segments overlap.

6. The hernia repair patch of claim 1 wherein the fabric material (20) is folded over and secured to the ends of the wire segments (12).

7. The hernia repair patch of claim 1 wherein the shape memory alloy comprises NiTi with a percentage of Ni in the alloy range of from 49 to 51 atomic percent.

8. The hernia repair patch of claim 1 wherein the shape memory alloy exhibits a transformation temperature of about 37° C.

9. The hernia repair patch of any one of claims 1, 7. and 8 in which the prosthetic fabric material (20) is selected from a group consisting of a woven mesh of polypropylene fabrics and expanded PTFE.

10. The hernia repair patch of claim 1 wherein prosthetic fabric material (20) is generally planar and has a major longitudinal dimension greater in length than a length of a minor transverse dimension.

11. The hernia repair patch of claim 1 wherein the prosthetic fabric material (20) has a generally rectangular shape with rounded corners.

12. The hernia repair patch of claim 11 wherein the wire frame (12, 14) is attached to the prosthetic fabric material (20) so that the patch has a generally rectangular shape with rounded corners when the wire frame is in an austenite form and a compact shape of low profile when in a martenistic form.

13. The hernia repair patch of claim 1 wherein the attach points at the vertices (18) are formed by bending the wire segments to form a u-shaped apex (34).

14. The hernia repair patch of claim 1 wherein the wire frame (12, 14) is in a relaxed state when the wire frame is attached to the fabric material (20).

15. The hernia repair patch of claim 1 wherein the wire frame (12, 14) is compressed upon being attached to the fabric material, and returns to a more relaxed state after said wire frame is attached to the fabric material, thereby applying a stretching force to the fabric material.

## Patentansprüche

1. Hernie-Reparatur-Patch (10), umfassend:
(a) ein Drahtgerüst (12, 14), das aus einer Formgedächtnislegierung gebildet ist, wobei das Drahtgerüst zwei überlappende v-förmige Drahtsegmente (12, 14) enthält, von welchen jedes Seiten ungleicher Länge aufweist, deren Scheitelpunkte (18) in entgegen gesetzten Richtungen angeordnet sind, wobei die Drahtsegmente Befestigungspunkte (16) aufweisen, die an freien Enden jedes der Drahtsegmente und an deren Scheitelpunkten angeordnet sind, wobei die Befestigungspunkte, die an den freien Enden angeordnet sind, geschlossene Schlingen umfassen;
(b) ein prothetisches Gewebematerial (20), das an dem Gerüst befestigt ist und von diesem gestützt wird, wobei Nähte (28, 30) das Gewebe an dem Drahtgerüst an den Befestigungspunkten halten, wobei das Drahtgerüst eine Dehnungskraft auf das Gewebematerial ausübt.

2. Hernie-Reparatur-Patch nach Anspruch 1, wobei die Befestigungspunkte an den Scheitelpunkten (18) durch Biegen der Drahtsegmente (12, 14) zur Bildung der Schlingen gebildet sind.

3. Hernie-Reparatur-Patch nach Anspruch 2, wobei jede Schlinge etwa 540 Grad ist.

4. Hernie-Reparatur-Patch nach Anspruch 1, wobei der Winkel zwischen den zwei Seiten der v-förmigen DrahtSegmente (12, 14) ungefähr zwischen 90 und 120 Grad im entspannten Zustand beträgt und auf etwa 30 Grad zusammengepresst ist, wenn der Draht an dem Gewebematerial befestigt ist.

5. Hernie-Reparatur-Patch nach Anspruch 1, wobei Schlingen (32) eines Fadens um die Drahtsegmente (12, 14) angeordnet sind, wo die Drahtsegmente überlappen.

6. Hernie-Reparatur-Patch nach Anspruch 1, wobei das Gewebematerial (20) über die Enden der Drahtsegmente (12) gefaltet und an diesen befestigt ist.

7. Hernie-Reparatur-Patch nach Anspruch 1, wobei die Formgedächtnislegierung NiTi mit einem Prozentsatz von Ni in der Legierung in einem Bereich von 49 bis 51 Atomprozent umfasst.

8. Hernie-Reparatur-Patch nach Anspruch 1, wobei die Formgedächtnislegierung eine Transformationstemperatur von etwa 37°C aufweist.

9. Hernie-Reparatur-Patch nach einem der Ansprüche 1, 7 und 8, wobei das prothetische Gewebematerial (20) ausgewählt ist aus der Gruppe bestehend aus einem gewobenen Geflecht aus Polypropylenstoffen und expandiertem PTFE.

10. Hernie-Reparatur-Patch nach Anspruch 1, wobei das prothetische Gewebematerial (20) im Allgemeinen eben ist und eine Längenhauptdimension aufweist, deren Länge größer als eine Länge einer Quernebendimension ist.

11. Hernie-Reparatur-Patch nach Anspruch 1, wobei das prothetische Gewebematerial (20) eine im Allgemeinen rechteckige Form mit abgerundeten Ecken hat.

12. Hernie-Reparatur-Patch nach Anspruch 11, wobei das Drahtgerüst (12, 14) an dem prothetischen Gewebematerial (20) so befestigt ist, dass das Patch eine im Allgemeinen rechteckige Form mit abgerundeten Ecken hat, wenn das Drahtgerüst in einer Austenitform ist, und eine kompakte Form geringen Profils, wenn es in einer martensitischen Form ist.

13. Hernie-Reparatur-Patch nach Anspruch 1, wobei die Befestigungspunkte an den Scheitelpunkten (18) durch Biegen der Drahtsegmente zur Bildung einer u-förmigen Spitze (34) gebildet sind.

14. Hernie-Reparatur-Patch nach Anspruch 1, wobei das Drahtgerüst (12, 14) in einem entspannten Zustand ist, wenn das Drahtgerüst an dem Gewebematerial (20) befestigt ist.

15. Hernie-Reparatur-Patch nach Anspruch 1, wobei das Drahtgerüst (12, 14) bei der Befestigung an dem Gewebematerial zusammengepresst wird und in einen entspannteren Zustand zurückkehrt, nachdem das Drahtgerüst an dem Gewebematerial befestigt ist, wodurch eine Dehnungskraft auf das Gewebematerial ausgeübt wird.

## Revendications

1. Pièce de traitement chirurgical de la hernie (10) comprenant :
(a) un cadre de fil métallique (12, 14) formé d'un alliage à mémoire de forme dans lequel le cadre de fil métallique comprend deux segments chevauchants de fil métallique en V (12, 14) comprenant chacun des côtés inégaux en longueur et des sommets (18) disposés dans des sens opposés, où les segments de fil métallique ont des points de fixation (16) disposés sur des extrémités libres de chacun des segments de fil métallique et en leurs sommets, les points de fixation disposés sur les extrémités libres comprenant des boucles fermées ;
(b) un matériau de tissu prothétique (20) fixé à et supporté par ledit cadre dans lequel des points de suture (28, 30) maintiennent le tissu au cadre de fil métallique au niveau desdits points de fixation, où le cadre de fil métallique applique une force d'étirement au matériau de tissu.

2. Pièce de traitement chirurgical de la hernie selon la revendication 1, où les points de fixation aux sommets (18) sont formés par flexion des segments de fil métallique (12, 14) pour former lesdites boucles.

3. Pièce de traitement chirurgical de la hernie selon la revendication 2, où chaque boucle est d'environ 540 degrés.

4. Pièce de traitement chirurgical de la hernie selon la revendication 1, dans laquelle l'angle entre les deux côtés des segments de fil métallique en V (12, 14) est approximativement compris entre 90 et 120 degrés lorsqu'il est dans un état détendu et comprimé à environ 30 degrés lorsque le fil métallique est fixé au matériau de tissu.

5. Pièce de traitement chirurgical de la hernie selon la revendication 1, dans laquelle des boucles (32) de fil sont placées autour des segments de fil métallique (12, 14) où les segments de fil métallique se chevauchent.

6. Pièce de traitement chirurgical de la hernie selon la revendication 1, dans laquelle le matériau de tissu (20) est replié sur les et fixé aux extrémités des segments de fil métallique (12).

7. Pièce de traitement chirurgical de la hernie selon la revendication 1, dans laquelle l'alliage à mémoire de forme comprend du NiTi avec un pourcentage de Ni dans l'alliage de 49 à 51 pour cent atomique.

8. Pièce de traitement chirurgical de la hernie selon la revendication 1, dans laquelle l'alliage à mémoire de forme présente une température de transformation d'environ 37 °C.

9. Pièce de traitement chirurgical de la hernie selon l'une quelconque des revendications 1, 7 et 8, dans laquelle le matériau de tissu prothétique (20) est choisi dans un groupe constitué d'une maille tissée de tissu de polypropylène et de PTFE expansé.

10. Pièce de traitement chirurgical de la hernie selon la revendication 1, dans laquelle le matériau de tissu prothétique (20) est globalement plan et a une dimension longitudinale majeure plus grande en longueur qu'une longueur d'une dimension transversale mineure.

11. Pièce de traitement chirurgical de la hernie selon la revendication 1, dans laquelle le matériau de tissu prothétique (20) a une forme globalement rectangulaire avec des coins arrondis.

12. Pièce de traitement chirurgical de la hernie selon la revendication 11, dans laquelle le cadre de fil métallique (12, 14) est fixé au matériau de tissu prothétique (20) de sorte que la pièce a une forme globalement rectangulaire avec des coins arrondis lorsque le cadre de fil métallique est sous une forme austénitique et une forme compacte de bas profil lorsqu'il est sous une forme martensitique.

13. Pièce de traitement chirurgical de la hernie selon la revendication 1, dans laquelle les points de fixation aux sommets (18) sont formés par flexion des segments de fil métallique pour former un sommet en U (34).

14. Pièce de traitement chirurgical de la hernie selon la revendication 1, dans laquelle le cadre de fil métallique (12, 14) est dans un état détendu lorsque le cadre de fil métallique est fixé au matériau de tissu (20).

15. Pièce de traitement chirurgical de la hernie selon la revendication 1, dans laquelle le cadre de fil métallique (12, 14) est comprimé lors de la fixation au matériau de tissu, et retourne à un état plus détendu après la fixation dudit cadre de fil métallique au matériau de tissu, appliquant ainsi une force d'étirement au matériau de tissu.
